# EUROPEAN PATENT APPLICATION

(11) **EP 4 764 501 A1**
(43) Date of publication of application: **24.06.2026**
(21) Application number: 23948806.7
(22) Date of filing: 14.08.2023
(51) Int. Cl.: G01N 33/68

(54) **COMPOSITION, KIT, AND METHOD FOR DETECTING LARGE-SIZE CTNITC, AND USE THEREOF**

(71) Applicant: Shenzhen Mindray Bio-Medical Electronics Co., Ltd., Shenzhen, Guangdong 518057 (CN)
(72) Inventor: ZHANG, Yi, Shenzhen, Guangdong 518057 (CN); LIU, Yuqing, Shenzhen, Guangdong 518057 (CN); LIU, Junjun, Shenzhen, Guangdong 518057 (CN); KATRUKHA, Ivan, Moscow 117186 (RU); RIABKOVA, Natalia, Moscow 117186 (RU)
(74) Representative: KIPA AB
(86) International application number: PCT/CN2023/112994
(87) International publication number: WO 2025/035371

(57) **Abstract**

The present application relates to a composition, kit, and method for detecting a large-size cTnITC, and a use thereof. Specifically, the present application relates to a composition and kit containing an antibody specifically binding to any segment of an amino acid sequence of cTnT from position 67 to position 222 and an antibody specifically binding to any segment of an amino acid sequence of TnC, a detection method, and a use in vitro diagnosis of myocardial injury.

## Description

### TECHNICAL FIELD

The disclosure relates to the field of immunodetection, and in particular relates to a composition and a kit for detecting a large-size cTnITC (large-size cardiac troponin ternary complex), a detection method, and the use thereof in the *in-vitro* diagnosis of myocardial injury.

### BACKGROUND

Cardiac troponin (cTn) is a specific marker for myocardial injury and may be used to detect myocardial injury after or during myocardial infarction. Troponin is composed from three subunits, namely cardiac troponin I (cTnl), cardiac troponin T (cTnT) and troponin C (TnC). The concentrations of cTnl and cTnT in serum are highly correlated with the severity of myocardial injury, and are recommended by the American and European Cardiology Societies as highly specific and sensitive markers for myocardial infarction. However, numerous factors induce elevated cardiac troponin concentrations in the blood, such as pulmonary embolism, acute or chronic heart failure, cardiac trauma, endocarditis and myocarditis, in addition to myocardial infarction. This reduces the clinical specificity of cTn for myocardial infarction. Therefore, a combination with other methods is typically required for the diagnosis of myocardial infarction.

Cardiac troponin typically binds to actin filaments in the form of a ternary complex cTnITC. In the event of myocardial injury, troponin dissociates from myofilaments and is released into the bloodstream. Intracellularly or in the circulating blood, full-length cTnITC undergoes proteolytic degradation, gradually forming low-molecular-weight ternary complexes (LMW-cTnITC), binary complexes (cTnIC) and free cTnT. Studies have shown that the existing forms of troponin in the blood are associated with the physiological and pathological status of individuals. Detection of different forms of cTnITC complex helps physicians to gain a more comprehensive understanding of the physiological and pathological status of patients, thereby aiding in rapid and accurate diagnosis and guiding patient prognosis.

Currently, the traditional immunological methods for cardiac troponin detection mainly include enzyme-linked immunosorbent assay (ELISA), gold immunochromatographic assay (GICA), electrochemiluminescence (ECL), chemiluminescence immunoassay (CLIA), and other technologies. The chemiluminescence immunoassay technology has high sensitivity, strong specificity, a wide linear range, good operability and high degree of automation, and is the immunological assay method with the broadest clinical application prospects. Based on the chemiluminescence immunoassay technology, existing high-sensitivity cardiac troponin detection methods are typically used to detect cTnl and cTnT, but cannot distinguish between troponin complexes and free troponin. It has been reported in the literature that a double-antibody sandwich immunoassay for cTnITC complexes is achieved by using capture antibodies that recognize the epitopes of the binary TnIC complex and detection antibodies that recognize the TnT antigen. This detection method has a low signal-to-noise ratio and insufficient specificity, making it difficult to achieve rapid, sensitive and specific detection of the cTnITC complex.

### SUMMARY

An object of the disclosure is to provide a technical solution capable of detecting a specific form of cTnITC in a sample with high sensitivity and rapid speed. By means of the cooperation of a capture antibody and a detection antibody, the specific detection is achieved. In particular, the addition of an antibody that specifically binds to TnC to the capture antibody or detection antibody is very beneficial for increasing the signal-to-noise ratio of the detection system and improving the detection sensitivity.

In a first aspect, the disclosure provides a composition for detecting a large-size cTnITC in a sample, comprising a first group of antibodies and a second group of antibodies, wherein, the first group of antibodies comprises one or more antibody 1, each being independently selected from antibodies that specifically bind to any one segment of the amino acid sequence of positions 67-222 of cTnT;
the second group of antibodies comprises one or more antibody 2, each being independently selected from antibodies that specifically bind to any one segment of the amino acid sequence of TnC.

In a second aspect, the disclosure provides a kit for detecting a large-size cTnITC in a sample, comprising a capture antibody and a detection antibody, wherein the capture antibody is selected from one group of a first group of antibodies and a second group of antibodies, and the detection antibody is selected from the other group of the first group of antibodies and the second group of antibodies, wherein,
the first group of antibodies comprises one or more antibody 1, each being independently selected from antibodies that specifically bind to any one segment of the amino acid sequence of positions 67-222 of cTnT;
the second group of antibodies comprises one or more antibody 2, each being independently selected from antibodies that specifically bind to any one segment of the amino acid sequence of TnC.

In a third aspect, the disclosure provides a method for detecting a large-size cTnITC in a sample *in vitro,* comprising steps of:
obtaining a test sample;
contacting the test sample with a capture antibody to form an antibody-antigen complex;
contacting the antibody-antigen complex with a detection antibody bearing a detectable label to form an antibody-antigen-antibody complex; and
detecting the signal produced by the detectable label to determine the presence and/or content of the large-size cTnITC in the test sample,
wherein the capture antibody is selected from one group of a first group of antibodies and a second group of antibodies, and the detection antibody is selected from the other group of the first group of antibodies and the second group of antibodies:
   the first group of antibodies comprises one or more antibody 1, each being independently selected from antibodies that specifically bind to any one segment of the amino acid sequence of positions 67-222 of cTnT;
   the second group of antibodies comprises one or more antibody 2, each being independently selected from antibodies that specifically bind to any one segment of the amino acid sequence of TnC.

In a fourth aspect, the disclosure provides the use of the composition according to any of the first aspect in the preparation of a reagent for detecting a large-size cTnITC.

In a fifth aspect, the disclosure provides the use of the composition according to any of the first aspect, the kit according to any of the second aspect, or the method according to any of the third aspect in the *in-vitro* diagnosis of myocardial injury.

In a sixth aspect, the disclosure provides a method for diagnosing myocardial injury *in vitro,* comprising a step of detecting a large-size cTnITC in a sample from a subject using the composition according to any of the first aspect, the kit according to any of the second aspect, or the method according to any of the third aspect.

### BRIEF DESCRIPTION OF THE DRAWINGS

The drawings illustrated herein are intended to provide a further understanding of the disclosure and form a part hereof, and exemplary embodiments of the disclosure and the description thereof are intended to explain the disclosure and are not to be construed as unduly limiting the disclosure. In the figures:
FIG. 1 shows the signal-to-noise ratio analysis of large-size cTnITC detection kits;
FIG. 2 shows the verification of specificity of large-size cTnITC detection kits using serum samples;
FIG. 3 shows the linear analysis of large-size cTnITC detection kit 3;
FIG. 4 shows the linear analysis of large-size cTnITC detection kit 4;
FIG. 5 shows the signal-to-noise ratio analysis of large-size cTnITC detection kit 3 and detection kit 3a;
FIG. 6 shows the detection signal-to-noise ratio analysis of large-size cTnITC detection kit 3 and detection kit 3a in clinical serum samples;
FIG. 7 shows the verification of specificity of large-size cTnITC detection kit 3a using serum samples;
FIG. 8 shows the analysis of trends of change in large-size cTnITC detection using large-size cTnITC detection kit 3, and in high-sensitivity cTnl and cTnT detection using existing kits; and
FIG. 9 shows the analysis of rates of change in concentrations of a large-size cTnITC detection using large-size cTnITC detection kit 3, and of high-sensitivity cTnl and cTnT detection using existing kits.

### DESCRIPTION OF THE PREFERRED EMBODIMENTS

The technical solutions in the embodiments will be described below clearly and completely in conjunction with the accompanying drawings. Obviously, the embodiments described are merely some rather than all of the embodiments of the disclosure. The following description of embodiments is merely illustrative and in no way construed as limiting the disclosure. On the basis of the embodiments, all the other embodiments that would have been obtained by those of ordinary skill in the art without any inventive effort shall fall within the scope of protection of the disclosure.

Herein, unless otherwise stated, the scientific and technical terms used have the meanings commonly understood by those skilled in the art. Moreover, the immunological laboratory procedures used herein are conventional procedures widely used in the respective fields. Meanwhile, in order to facilitate a better understanding of the embodiments of the disclosure, definitions and explanations of relevant terms are provided below.

As used herein, the terms "comprise", "include" or any other variation thereof are intended to cover non-exclusive inclusion, so that a method or an apparatus comprising a series of elements comprises not only explicitly recorded elements, but also other elements not explicitly listed, or elements inherent in implementing the method or apparatus. In the absence of more restrictions, the element defined by the phrase "comprise a/an..." does not exclude another related element in a method or device that includes the element.

As used herein, the term "at least one" refers to one or more than one under reasonable conditions, for example, two, three, four, five, or ten, etc.

As used herein, the terms "first" and "second" are only used to distinguish similar objects, and do not represent a specific order for the objects. It may be understood that "first" and "second" may be interchanged in a specific order or sequence where permissible. It should be understood that the objects distinguished by "first" and "second" may be interchanged where appropriate, so that the embodiments of the disclosure described herein can be implemented in an order other than those illustrated or described herein.

As used herein, the term "specific binding" refers to a non-random binding reaction between two molecules (i.e., binding molecule and target molecule), such as a reaction between an antibody and the antigen to which it is directed. The binding affinity between the two molecules can be described by the KD value. The KD value refers to the dissociation constant derived from the ratio of kd (the dissociation rate of a specific binding molecule-target molecule interaction; also referred to as koff) to ka (the association rate of a specific binding molecule-target molecule interaction; also referred to as kon), or expressed as kd/ka in molar concentration (M). The smaller the KD value, the tighter the binding between the two molecules and the higher the affinity. In certain embodiments, an antibody that specifically binds to an antigen (or an antibody specific for an antigen) refers to an antibody that binds to the antigen with a KD of less than about 10⁻⁵ M, for example, less than about 10⁻⁶ M, 10⁻⁷ M, 10⁻⁸ M, 10⁻⁹ M or 10⁻¹⁰ M or less. Corresponding methods for analyzing antibody specificity are described, for example, in the following references: Harlow & Lane (1988) Antibodies: A Laboratory Manual, Cold Spring Harbor Laboratory Press, and Harlow & Lane (1999) Using Antibodies: A Laboratory Manual, Cold Spring Harbor Laboratory Press. Non-limiting examples of suitable studies include, for example, binding studies, and blocking and competition studies performed with molecules that are structurally and/or functionally closely related. These studies can be performed using the following methods, for example, fluorescence-activated cell sorting (FACS) analysis, flow cytometry titration (FACS titration) analysis, surface plasmon resonance (SPR), isothermal titration calorimetry (ITC), fluorescence titration or radiolabeled ligand binding assay. Further methods include, for example, Western Blot, ELISA (including competitive ELISA) testing, RIA testing, ECL testing and IRMA testing.

As used herein, the term "troponin (Tn)" refers to a protein located on actin filaments within muscle cells that regulates the calcium-mediated interaction between actin and myosin. Troponin is present in cardiac and skeletal muscles and is composed of three subunits: troponin T (TnT), troponin I (Tnl) and troponin C (TnC). TnT is a tropomyosin-binding subunit that interacts with actin and tropomyosin; Tnl is an inhibitory subunit that inhibits the ATPase activity of actomyosin; and TnC is the only subunit that binds to calcium and can mediate the contraction of skeletal or cardiac muscles. The term "cardiac troponin (cTn)" refers to all troponin isoforms expressed in cardiac cells, preferably subendocardial cells. These isoforms have been well characterized in the art, for example, as described in Anderson 1995, Circulation Research, vol. 76, no. 4: 681-686 and Ferrieres 1998, Clinical Chemistry, 44: 487-493. The term "cardiac troponin" further includes variants of a specific cardiac troponin that have at least the same basic biological and immunological properties as the specific cardiac troponin. In particular, these variants are considered to share the same basic biological and immunological properties if they are detected by the same specificity as mentioned herein. It should be understood that troponin isoforms may be determined collectively (simultaneously or sequentially) or individually (i.e., without assaying any other isoforms whatsoever).

As used herein, the term "cardiac troponin T (cTnT)" refers to the cardiac troponin T subunit, the amino acid sequence of which is disclosed in the UniProt database under accession number P45379.

As used herein, the term "cardiac troponin I (cTnl)" refers to the cardiac troponin I subunit, the amino acid sequence of which is disclosed in the UniProt database under accession number P19429.

As used herein, the term "troponin C (TnC)" refers to the troponin C subunit, the amino acid sequence of which is disclosed in the UniProt database under accession number P63316.

As used herein, the terms "large-size cardiac troponin ternary complex" and "large-size cTnITC" are used interchangeably herein and are intended to encompass any complex formed by a full-length protein or fragment of TnC, a full-length protein or fragment of cTnl, one or more fragments of amino acid residues 223-287 of cTnT, and one or more fragments of amino acid residues 1-222 of cTnT.

As used herein, the term "antibody" has the meaning commonly understood in the art and refers to an immunoglobulin molecule that is generally composed of two pairs of polypeptide chains, each pair containing one light chain (LC) and one heavy chain (HC). Antibody light chains may be classified as κ (kappa) and λ (lambda) light chains. Heavy chains may be classified as µ, δ, y, α and ε heavy chains, which define the antibody isotypes as IgM, IgD, IgG, IgA and IgE, respectively. Each heavy chain is composed of a heavy chain variable region (VH) and a heavy chain constant region (CH). Each light chain is composed of a light chain variable region (VL) and a light chain constant region (CL). The light chain constant region is composed of a single domain CL. The constant domains are not directly involved in the binding of an antibody to an antigen, but exhibit a variety of effector functions. For example, these domains can mediate the binding of immunoglobulins to host tissues or factors, including various cells of the immune system (e.g., effector cells) and the first component of the classical complement system (C1q). The VH and VL regions may be further subdivided into hypervariable regions (referred to as complementarity-determining regions, CDRs), interspersed with more conserved regions referred to as framework regions (FRs). Each VH and VL is composed of three CDRs and four FRs arranged from the amino terminus to the carboxyl terminus in the following order: FR1, CDR1, FR2, CDR2, FR3, CDR3, and FR4. The variable regions (VH and VL) of each heavy/light chain pair form an antigen-binding site, respectively. The assignment of amino acids to each region or domain may follow the definitions described in Kabat, Sequences of Proteins of Immunological Interest (National Institutes of Health, Bethesda, Md. (1987 and 1991)), or Chothia & Lesk (1987), J. Mol. Biol. 196: 901-917; Chothia et al. (1989), Nature 342: 878-883. As used herein, unless otherwise clearly defined in the context, when referring to the term "antibody", it includes not only intact antibodies but also antigen-binding fragments of antibodies.

The disclosure is applicable to various detection systems including, but not limited to, fluorescence-labeled reaction systems, enzyme-linked immunosorbent assay (ELISA) systems, bioluminescent systems, etc.

### Detection reagent and method

An object of the disclosure is to provide a detection reagent capable of sensitive and specific detection of a large-size cTnITC (including the full-length cTnITC and moderate hydrolysis products, where the hydrolysis products still include cTnl, cTnT and TnC subunits or fragments thereof, and a TnT fragment at positions 67-222), without recognizing further hydrolyzed ternary complexes, binary complexes or free subunits and fragments thereof.

In particular, in a first aspect, the disclosure provides a composition for detecting a large-size cTnITC in a sample, which composition includes a first group of antibodies and a second group of antibodies, wherein
the first group of antibodies includes one or more antibody 1, each being independently selected from antibodies that specifically bind to any one segment of the amino acid sequence of positions 67-222 of cTnT;
the second group of antibodies includes one or more antibody 2, each being independently selected from antibodies that specifically bind to any one segment of the amino acid sequence of TnC.

On the basis of the first aspect, a second aspect of the disclosure provides a kit for detecting a large-size cTnITC in a sample, which kit includes a capture antibody and a detection antibody, wherein the capture antibody is selected from one group of a first group of antibodies and a second group of antibodies, and the detection antibody is selected from the other group of the first group of antibodies and the second group of antibodies, wherein
the first group of antibodies includes one or more antibody 1, each being independently selected from antibodies that specifically bind to any one segment of the amino acid sequence of positions 67-222 of cTnT;
the second group of antibodies includes one or more antibody 2, each being independently selected from antibodies that specifically bind to any one segment of the amino acid sequence of TnC.

That is, the capture antibody includes at least antibody 1 and the detection antibody includes at least antibody 2; alternatively, the capture antibody includes at least antibody 2 and the detection antibody includes at least antibody 1.

In the embodiments of the disclosure, the first group of antibodies does not comprise an antibody that specifically binds to any one segment of the amino acid sequence of positions 223-287 of cTnT.

In some embodiments, the capture antibody may be coated on the surface of a solid-phase carrier (e.g., magnetic beads, latex particles, microplates, plastic beads, plastic tubes, immunochromatographic test strips or detection cards) to capture an antigen in a sample. Subsequently, a detection antibody bearing a detectable label is allowed to bind to the capture antibody coated on the surface of the solid-phase carrier, and the content of the antigen in the sample is determined by measuring the amount of the detectable label on the bound detection antibody.

The detectable label may be any substance detectable by fluorescent, spectroscopic, photochemical, biochemical, immunological, electrical, optical or chemical means. Particularly preferably, such labels are suitable for immunological detection (e.g., enzyme-linked immunoassay, radioimmunoassay, fluorescence immunoassay, chemiluminescent immunoassay, etc.). Such labels are well known in the art and include, but are not limited to, enzymes (e.g., horseradish peroxidase, alkaline phosphatase, β-galactosidase, urease, glucose oxidase, etc.), radionuclides (e.g., 3H, 125I, 35S, 14C or 32P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7 and Alexa 750)), chemiluminescent substances (such as acridine ester compounds), and biotin for binding to avidin (e.g., streptavidin) modified with the above labels. The labels encompassed in the disclosure may be detected by methods known in the art. For example, radiolabels may be detected using photographic film or a scintillation counter, and fluorescent markers may be detected using a light detector to detect the emitted light. Enzyme labels are typically detected by providing the enzyme with a substrate and detecting the reaction products produced by the action of the enzyme on the substrate. Chemiluminescent substances (e.g., acridinium ester compounds) are typically detected by providing an excitation solution and/or a catalyst to the luminescent substance to measure the emitted light. Biotin is typically detected by providing avidin (e.g., streptavidin) modified with the above labels for the biotin and detecting the labels carried by the avidin bound to the biotin. In certain embodiments, the detectable labels as described above may be linked to the antibody or the antigen-binding fragment thereof of the disclosure via a linker of different lengths to reduce potential steric hindrance. In some embodiments, the detectable labels are selected from fluoresceins, chemiluminescent substances (e.g., acridinium ester compounds), enzymes (e.g., horseradish peroxidase or alkaline phosphatase), radioisotopes, biotin, colloidal gold and magnetic particles.

In some embodiments, the kit may further include a reagent for enabling the detection of a corresponding detectable label (a substrate for the detectable label). For example, when the detectable label is an enzyme, the kit may further include a chromogenic substrate for the corresponding enzyme, such as o-phenylenediamine (OPD), tetramethylbenzidine (TMB), ABTS or luminol compounds for horseradish peroxidase, or p-nitrophenyl phosphate (p-NPP) or AMPPD for alkaline phosphatase. For example, when the detectable label is a chemiluminescent reagent (e.g., acridinium ester compound), the kit may further include a pre-excitation solution and/or an excitation solution for chemiluminescence.

The kit of the disclosure is particularly suitable for immunoassays, such as ELISA, and particularly the double-antibody sandwich chemiluminescent immunoassay.

In the disclosure, the amount of a large-size cTnITC may be detected directly or indirectly. The amount or concentration of the detection antibody may be directly detected on the basis of the detectable label signal thereof, which signal is directly correlated with the target antigen in the sample. Such signals are sometimes referred to herein as intensity signals. For example, such signals may be obtained by measuring the intensity values of the specific physical or chemical properties of the detection antibody. Indirect measurements include measurement of signals from secondary components (i.e., not the detection antibody itself) or biological readout systems, such as measurable cellular responses, ligands, labels or enzymatic reaction products.

In a third aspect, the disclosure provides a method for detecting a large-size cTnITC in a sample *in vitro,* which method includes the steps of:
obtaining a test sample;
contacting the test sample with a capture antibody to form an antibody-antigen complex;
contacting the antibody-antigen complex with a detection antibody bearing a detectable label to form an antibody-antigen-antibody complex; and
detecting the signal produced by the detectable label to determine the presence and/or content of the large-size cTnITC in the test sample,
wherein the capture antibody is selected from one group of a first group of antibodies and a second group of antibodies, and the detection antibody is selected from the other group of the first group of antibodies and the second group of antibodies:
   the first group of antibodies includes one or more antibody 1, each being independently selected from antibodies that specifically bind to any one segment of the amino acid sequence of positions 67-222 of cTnT;
   the second group of antibodies includes one or more antibody 2, each being independently selected from antibodies that specifically bind to any one segment of the amino acid sequence of TnC.

In the first aspect to the third aspect of the disclosure, as some embodiments, the second group of antibodies further includes:
one or more antibody 3, each being independently selected from antibodies that specifically bind to cTnIC; and/or
one or more antibody 4, each being independently selected from antibodies that specifically bind to any one segment of the amino acid sequence of positions 18-210 of cTnT.

Herein, by means of combining antibody 2 with an antibody that specifically recognizes the cTnIC binary complex or an antibody that specifically recognizes cTnl, the signal-to-noise ratio of the detection reagent can be significantly increased.

In the first aspect to the third aspect of the disclosure, as some embodiments, in some embodiments, the first group of antibodies includes one or more of the antibody 1, and the second group of antibodies includes one or more of the antibody 2 and one or more of the antibody 3.

In the first aspect to the third aspect of the disclosure, as some embodiments, the first group of antibodies includes at least two antibody 1. In some embodiments, the second group of antibodies includes at least two antibody 2. In some embodiments, the first group of antibodies includes at least two antibody 1; the second group of antibodies includes at least two antibody 2 and optionally at least two antibody 3. A combination of multiple antibodies that bind to the same target protein is beneficial for the detection of a large-size cTnITC.

The binding epitopes of the antibodies are screened in the disclosure. In some embodiments, each of the antibody 1 is independently selected from antibodies that specifically bind to amino acids at positions 67-86, positions 119-138, positions 132-151, positions 145-164 or positions 171-190 of cTnT. In some embodiments, each of the antibody 1 is independently selected from antibodies that specifically bind to amino acids at positions 119-138, positions 132-151 or positions 171-190 of cTnT. In some embodiments, each of the antibody 1 is independently selected from antibodies that specifically bind to amino acids at positions 119-138 or positions 132-151 of cTnT.

For the binding epitope of antibody 4, in some embodiments, each of the antibody 4 is independently selected from antibodies that specifically bind to amino acids at positions 1-15, positions 13-22, positions 18-22, positions 18-28, positions 18-35, positions 22-31, positions 22-40, positions 23-29, positions 24-40, positions 25-40, positions 26-35, positions 34-37, positions 41-49, positions 83-89, positions 86-90, positions 87-90, positions 117-126, p ositions 130-145, positions 169-178, positions 186-192, positions 190-196 or positions 195-209 of cTnl. In some embodiments, each of the antibody 4 is independently selected from antibodies that specifically bind to amino acids at positions 22-40, positions 41-49 or positions 83-89 of cTnl. In some embodiments, each of the antibody 4 is independently selected from antibodies that specifically bind to amino acids at positions 41-49 of cTnl.

In some embodiments, the capture antibody is the first group of antibodies and the detection antibody is the second group of antibodies. Studies have found that the detection of a large-size cTnITC demonstrates a higher signal-to-noise ratio when the first group of antibodies is used as the capture antibody and the second group of antibodies is used as the detection antibody.

In the disclosure, commercially available antibodies targeting specific epitopes may be selected, or antibodies in the disclosure that have specific binding activity for specific epitopes of a large-size cTnITC may be obtained by means of general technical means in the art. One exemplary embodiment includes obtaining a monoclonal antibody with specific binding activity by hybridoma technology, which specifically includes the steps of selection and preparation of two parental cell lines, cell fusion, selective culture and cloning of hybridoma cells, preparation of monoclonal antibodies, specificity identification, purification, etc. Further, the steps of epitope identification and screening may be included, such as the identification of antibody-binding epitopes using techniques such as ELISA or surface plasmon resonance (SPR).

### Use

Due to the low sensitivity of existing cTnITC detection kits, the use thereof in the diagnosis of myocardial injury is severely compromised. Clinically, cTnl and cTnT are also commonly selected as highly specific and highly sensitive markers for myocardial infarction. On the basis of the above-mentioned composition, kit and detection method, the disclosure enables the rapid, highly sensitive and highly specific detection of a large-size cTnITC, which facilitates an in-depth understanding of the correlation between troponin and myocardial injury. Studies have shown that a large-size cTnITC is more sensitive to myocardial injury than cTnl and cTnT, and thus can serve as a more sensitive marker for myocardial injury to aid in disease diagnosis and prognosis assessment.

In a fourth aspect, the disclosure provides the use of the composition according to any of the first aspect in the preparation of a reagent for detecting a large-size cTnITC.

In a fifth aspect, the disclosure provides the use of the composition according to any of the first aspect, the kit according to any of the second aspect, or the method according to any of the third aspect in the *in-vitro* diagnosis of myocardial injury.

In a sixth aspect, the disclosure provides a method for diagnosing myocardial injury *in vitro,* which method includes a step of detecting a large-size cTnITC in a sample from a subject using the composition according to any of the first aspect, the kit according to any of the second aspect, or the method according to any of the third aspect.

In some embodiments, the sample is a bodily fluid or tissue sample from a subject, such as whole blood, serum, plasma (including heparin lithium plasma and EDTA plasma), urine, saliva, or biological tissue or cells, preferably whole blood, serum or plasma.

### Beneficial Effects

1) The disclosure achieves the rapid, highly sensitive and highly specific detection of a large-size cTnITC. By means of selecting an antibody that specifically binds to TnC as a capture antibody or a detection antibody, the signal-to-noise ratio of the system can be significantly increased, and the sensitivity can be improved.
2) Since the existing forms of troponin in the blood are associated with the physiological and pathological status of an individual, the specific detection of a large-size cTnITC facilitates an in-depth understanding of the correlation between troponin and diseases. The experimental results show that the large-size cTnITC exhibits a consistent trend of change with cTnT and cTnl, and thus has clinical value. Moreover, the concentration of the large-size cTnITC exhibits a faster rate of change, is more sensitive to myocardial injury in patients, and thus can serve as a more sensitive myocardial injury marker to aid in disease diagnosis and prognosis assessment.
3) The composition or kit of the disclosure can be used in conjunction with a fully automated chemiluminescence analyzer, which enables automated, fast and high-throughput detection of a large-size cTnITC.
4) The kit of the disclosure has high sensitivity, excellent specificity and good reproducibility.

### Examples

### Example 1 Detection Method and Construction of Detection Kits

The capture and detection antibodies were applied to a double-antibody sandwich chemiluminescence immunoassay, and detection kits were constructed for detecting a large-size cTnITC in a sample.

### (1) Large-size cTnITC detection kit 1

Capture antibody: antibody 1: antibody 329cc that specifically binds to the amino acid fragment 119-138 of cTnT.

Detection antibody: antibody 3: antibody 20C6cc that specifically binds to the epitope of the cTnIC complex.

### (2) Large-size cTnITC detection kit 2

Capture antibody: antibody 1: antibody 329cc that specifically binds to the amino acid fragment 119-138 of cTnT.

Detection antibody: antibody 4: antibody 19C7cc that specifically binds to the amino acid fragment 41-49 of cTnl.

### (3) Large-size cTnITC detection kit 3

Capture antibody: antibody 1: antibody 329cc that specifically binds to the amino acid fragment 119-138 of cTnT.

Detection antibody: antibody 2: antibody 7B9cc that specifically binds to TnC; and antibody 3: antibody 20C6cc that specifically binds to an epitope of the cTnIC complex.

### (4) Large-size cTnITC detection kit 4

Capture antibody: antibody 1: antibody 329cc that specifically binds to the amino acid fragment 119-138 of cTnT.

Detection antibody: antibody 2: antibody 7B9cc that specifically binds to TnC; and antibody 4: antibody 19C7cc that specifically binds to the amino acid fragment 41-49 of cTnl.

### (5) Large-size cTnITC detection kit 5

Capture antibody: antibody 1: antibody 329cc that specifically binds to the amino acid fragment 119-138 of cTnT.

Detection antibody: antibody 2: antibody 7B9cc that specifically binds to TnC.

### (6) Large-size cTnITC detection kit 6

Capture antibody: antibody 1: antibody 1C11cc that specifically binds to the amino acid fragment 171-190 of cTnT.

Detection antibody: antibody 2: antibody 7B9cc that specifically binds to TnC; and antibody 3: antibody Tcom8 that specifically binds to an epitope of the cTnIC complex.

### (7) Large-size cTnITC detection kit 7

Capture antibody: antibody 1: antibody 406cc that specifically binds to the amino acid fragment 132-151 of cTnT.

Detection antibody: antibody 2: antibody 7B9cc that specifically binds to TnC; and antibody 3: antibody 20C6cc that specifically binds to an epitope of the cTnIC complex.

### (8) Large-size cTnITC detection kit 8

Capture antibody: antibody 1: antibody 300cc that specifically binds to the amino acid fragment 119-138 of cTnT.

Detection antibody: antibody 2: antibody 7B9cc that specifically binds to TnC; and antibody 3: antibody 20C6cc that specifically binds to an epitope of the cTnIC complex.

In the experiments, kits obtained by replacing antibody 1, used in the large-size cTnITC detection kits 3-8, with the following antibodies were also used: antibody 7F4 or 7G7 with a specific binding site at the amino acid fragment 67-86 of cTnT, and antibody 2F3, 1A11 or 1F11cc with a specific binding site at the amino acid fragment 145-164 of cTnT. In the experiments, kits obtained by replacing antibody 4, used in the large-size cTnITC detection kit 4, with the following antibodies were also used: antibody M18cc with a specific binding site at the amino acid fragment 18-28 of cTnl, antibody 16A11cc, 16A12cc or 8E10cc with a specific binding site at the amino acid fragment 86-90 of cTnl, antibody M46 with a specific binding site at the amino acid fragment 130-145 of cTnl, and antibody MF4cc with a specific binding site at the amino acid fragment 190-196 of cTnl. The antibodies herein were purchased from HyTest Biotech (Shanghai) Co., Ltd.

The large-size cTnITC detection kits include:
A. Magnetic bead coating working solution, for the capture of a large-size cTnITC in a sample. The magnetic bead coating working solution includes: a mixture of superparamagnetic microparticles coated with capture antibodies.
B. Enzyme conjugate working solution, for the detection of a large-size cTnITC antigen captured by superparamagnetic microparticles. The enzyme conjugate working solution includes: an alkaline phosphatase-labeled detection antibody.

The detection method for a large-size cTnITC was as follows:
Step 1: A sample was added to a reaction tube together with a magnetic bead coating working solution and an enzyme conjugate working solution. After incubation, the large-size cTnITC in the sample was bound to an antibody coated on the magnetic beads, and meanwhile, an antibody-alkaline phosphatase conjugate was bound to the large-size cTnITC in the sample. After the reaction was completed, the solid phase was placed in a magnetic field. The magnetic field attracted the magnetic beads. The substance bound to the solid phase was retained, and the unbound substances were washed away.
Step 2: A chemiluminescent substrate (3-(2-spiroadamantane)-4-methoxy-4-(3-phosphoryloxy)-phenyl-1,2-dioxetane, AMPPD) was added to the reaction tube, and decomposed by the alkaline phosphatase, with one phosphate group removed, to form an unstable intermediate product. The intermediate product underwent intramolecular electron transfer to produce a methyl m-oxybenzoate anion. When the methyl m-oxybenzoate anion in the excited state returned to the ground state, chemiluminescence was produced. The number of photons produced during the reaction was then measured using a photomultiplier tube. The number of photons produced was proportional to the concentration of the large-size cTnITC in the sample. The amount of an analyte in the sample was determined by a calibration curve.

The above large-size cTnITC detection kits could be designed for use in combination with Mindray fully automated chemiluminescence analyzers, including models such as CL2000i, CL6000i and CL8000i.

### Example 2 Signal-to-Noise Ratio Analysis of Large-size cTnITC Detection Kits

Samples containing different concentrations of antigens were prepared, including two high-concentration samples (high-value samples) and two low-concentration samples (low-value samples). The antigen was a recombinant cardiac troponin ternary complex (Hytest, 8ITCR). The samples were separately analyzed using detection kits 1-8. Meanwhile, the signal from the blank sample without antigen was recorded, and the signal-to-noise ratio was calculated.

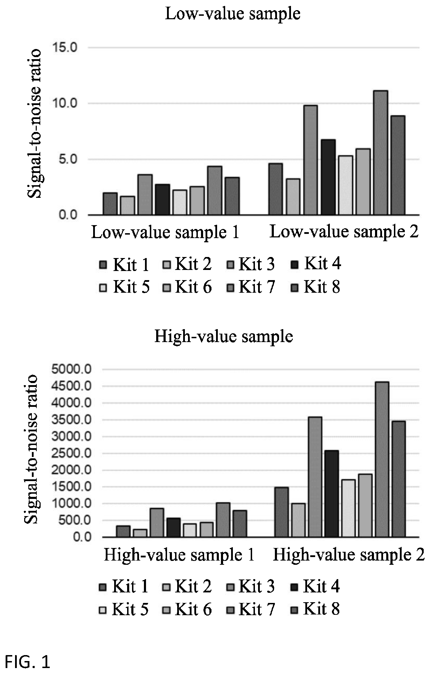

The test results are shown in FIG. 1. The signal-to-noise ratio of detection kit 3 was significantly higher than that of detection kit 1 and detection kit 5, and the signal-to-noise ratio of detection kit 4 was significantly higher than that of detection kit 2 and detection kit 5, indicating that the use of antibody 2 that specifically binds to TnC in combination with antibody 3 or antibody 4 was able to significantly increase the signal-to-noise ratio. Detection kits 3, 7 and 8 had a high signal-to-noise ratio, indicating that antibody 1 with specific binding sites at amino acids of cTnT at positions 119-138 and 132-151 significantly enhanced the signal-to-noise ratio.

In addition, replacing antibody 1 used in detection kits 3-8 with the following antibodies: antibody 7F4 or 7G7 with a specific binding site at the amino acid fragment 67-86 of cTnT, and antibody 2F3, 1A11 or 1F11cc with a specific binding site at the amino acid fragment 145-164 of cTnT, and replacing antibody 4 used in detection kit 4 with the following antibodies: antibody M18cc with a specific binding site at the amino acid fragment 18-28 of cTnl, antibody 16A11cc, 16A12cc or 8E10cc with a specific binding site at the amino acid fragment 86-90 of cTnl, antibody M46 with a specific binding site at the amino acid fragment 130-145 of cTnl, and antibody MF4cc with a specific binding site at the amino acid fragment 190-196 of cTnl, both effectively reflecting signal differences among samples.

### Example 3 Specificity Analysis of Large-size cTnITC Detection Kits

Equal concentrations of different antigens were spiked into the serum of healthy people, and the samples were separately analyzed using detection kits 1-8 by the chemiluminescent immunoassay method. The antigens analyzed included: cTnT (Hytest, 8RTT5), cTnl (Hytest, 8RT17), cTnIC (Hytest, 8ICR3) and cTnITC (Hytest, 8ITCR).

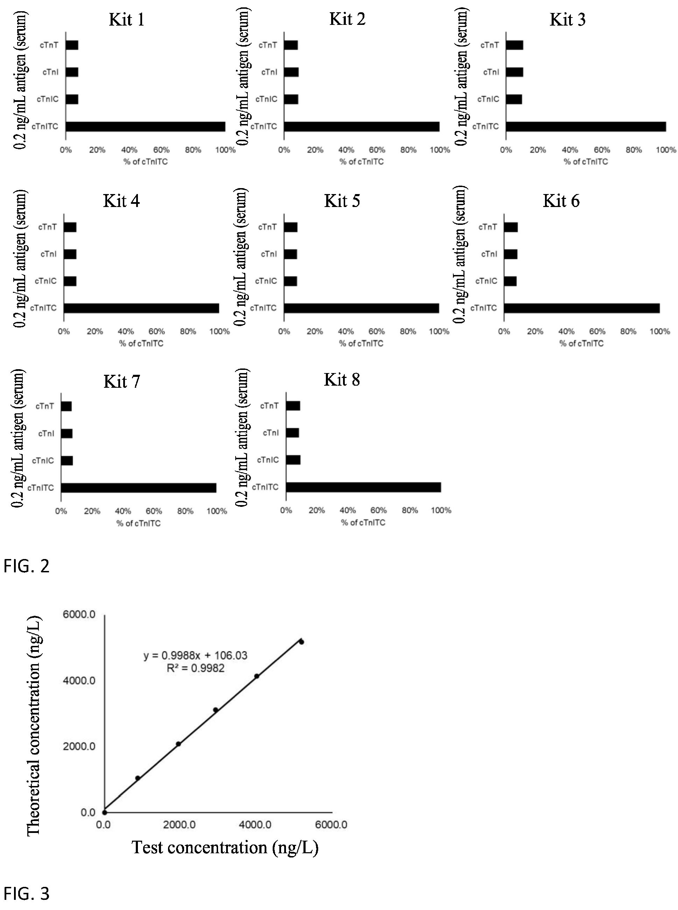

The experimental results are shown in FIG. 2. Detection kits 1-8 were all able to recognize only the cTnITC antigen, but were unable to recognize cTnT, cTnl or the binary cTnIC complex. In addition, replacing antibody 1 used in detection kits 3-8 with the following antibodies: antibody 7F4 or 7G7 with a specific binding site at the amino acid fragment 67-86 of cTnT, and antibody 2F3, 1A11 or 1F11cc with a specific binding site at the amino acid fragment 145-164 of cTnT, and replacing antibody 4 used in detection kit 4 with the following antibodies: antibody M18cc with a specific binding site at the amino acid fragment 18-28 of cTnl, antibody 16A11cc, 16A12cc or 8E10cc with a specific binding site at the amino acid fragment 86-90 of cTnl, antibody M46 with a specific binding site at the amino acid fragment 130-145 of cTnl, and antibody MF4cc with a specific binding site at the amino acid fragment 190-196 of cTnl, both effectively recognizing the cTnITC antigen.

### Example 4 Establishment of Limit of Blank and Limit of Detection for Large-size cTnITC Detection Kits

The limit of blank (LoB) and limit of detection (LoD) were established according to the recommendations of the Clinical and Laboratory Standards Institute (CLSI) (EP-17A2 Protocols for Determination of Limits of Detection and Limits of Quantitation).

The results of LoB test were derived from 5 blank samples, which were run over 4 days with 4 replicates per assay. General formula: LoB = mean + 1.65*SD.

The results of LoD test were derived from 5 low-concentration samples, which were run over 4 days with 4 replicates per assay. General formula: LoD = LoB + 1.65*SD.

The limit of blank and limit of detection were established for kits 1-8. The test results are shown in Table 1.

**Table 1: Limits of blank (LoB) and limits of detection (LoD) of the detection kits**

| Large-size cTnITC detection kit | 1 | 2 | 3 | 4 | 5 | 6 | 7 | 8 |
|---|---|---|---|---|---|---|---|---|
| Limits of blank (LoB) (ng/L) | 2.93 | 3.72 | 0.20 | 1.88 | 2.31 | 2.04 | 0.13 | 1.20 |
| Limit of detection (LoD) (ng/L) | 5.12 | 6.02 | 0.34 | 3.85 | 4.37 | 4.03 | 0.31 | 2.83 |

The limit of blank and limit of detection of detection kit 3 were significantly lower than those of detection kit 1 and detection kit 5, and the limit of blank and limit of detection of detection kit 4 were significantly lower than those of detection kit 2 and detection kit 5, indicating that the addition of antibody 2 that specifically binds to TnC and the use thereof in combination with antibody 3 or antibody 4 were able to significantly improve the sensitivity of the kits.

In addition, replacing antibody 1 used in detection kits 3-8 with the following antibodies: antibody 7F4 or 7G7 with a specific binding site at the amino acid fragment 67-86 of cTnT, and antibody 2F3, 1A11 or 1F11cc with a specific binding site at the amino acid fragment 145-164 of cTnT, and replacing antibody 4 used in detection kit 4 with the following antibodies: antibody M18cc with a specific binding site at the amino acid fragment 18-28 of cTnl, antibody 16A11cc, 16A12cc or 8E10cc with a specific binding site at the amino acid fragment 86-90 of cTnl, antibody M46 with a specific binding site at the amino acid fragment 130-145 of cTnl, and antibody MF4cc with a specific binding site at the amino acid fragment 190-196 of cTnl, both resulting in low LoB and LoD values.

### Example 5 Linear Analysis of Large-size cTnITC Detection Kits

Detection kit 3 and detection kit 4 were selected for linear analysis due to a high signal-to-noise ratio and a low limit of detection thereof.

Clinical serum samples were selected as high-concentration samples. The high-concentration samples were diluted proportionally to produce a series of diluted samples with a concentration range of 0-6000 ng/L.

The samples were separately analyzed using detection kit 3 by the chemiluminescent immunoassay method. The average of the test concentration results was linearly fitted against the theoretical concentration, and the correlation coefficient within the linear range was calculated.

The experimental results are shown in FIG. 3. The test concentrations of the diluted samples exhibited a linear relationship with the theoretical concentration. The R2 value in the linear range (0-6000 ng/L) was 0.9982.

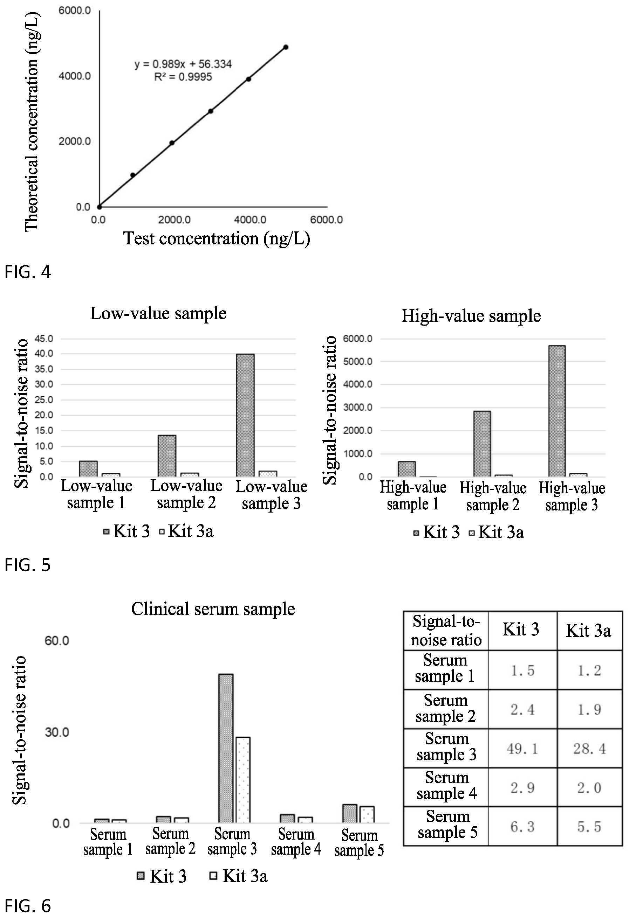

The samples were separately analyzed using detection kit 4 by the chemiluminescent immunoassay method. The average of the test concentration results was linearly fitted against the theoretical concentration, and the correlation coefficient within the linear range was calculated. The experimental results are shown in FIG. 4. The test concentrations of the diluted samples exhibited a linear relationship with the theoretical concentration. The R2 value in the linear range (0-6000 ng/L) was 0.9995.

### Example 6 Construction and Testing of Detection Kit with Capture Antibody and Detection Antibody Exchanged

Detection kit 3a was constructed by exchanging the capture antibody and the detection antibody of detection kit 3.

Detection kit 3 includes:
Capture antibody: antibody 1: antibody 329cc that specifically binds to the amino acid fragment 119-138 of cTnT.
Detection antibody: antibody 2: antibody 7B9cc that specifically binds to TnC; and antibody 3: antibody 20C6cc that specifically binds to an epitope of the cTnIC complex.

Detection kit 3a includes:
Capture antibody: antibody 2: antibody 7B9cc that specifically binds to TnC; and antibody 3: antibody 20C6cc that specifically binds to an epitope of the cTnIC complex.
Detection antibody: antibody 1: antibody 329cc that specifically binds to the amino acid fragment 119-138 of cTnT.

Samples containing different concentrations of antigens were prepared, including three high-concentration samples and three low-concentration samples. The antigen was a recombinant cardiac troponin ternary complex (Hytest, 8ITCR). The samples were separately analyzed using detection kits 3 and 3a. Meanwhile, the signal from the blank sample without antigen was recorded, and the signal-to-noise ratio was calculated. The test results are shown in FIG. 5.

Clinical serum samples from patients with cardiovascular diseases were separately analyzed using detection kits 3 and 3a. Meanwhile, the signals from serum samples of healthy people were recorded, and the signal-to-noise ratio was calculated. The test results are shown in FIG. 6. The signal-to-noise ratio of detection kit 3 was significantly higher than that of detection kit 3a, indicating that when a preferred combination of capture antibody and detection antibody was as follows: capture antibody: antibody 1 (which specifically binds to the amino acid fragment 119-138 of cTnT), and detection antibody: antibody 2 (which specifically binds to TnC) and antibody 3 (which specifically binds to an epitope of the cTnIC complex), the signal-to-noise ratio of the detection kit with the combination of capture antibody and detection antibody was higher. The signal-to-noise ratio of detection kit 3a was lower than that of detection kit 3, while the variation pattern of signal differences of detection kit 3a across different samples was similar to that of detection kit 3.

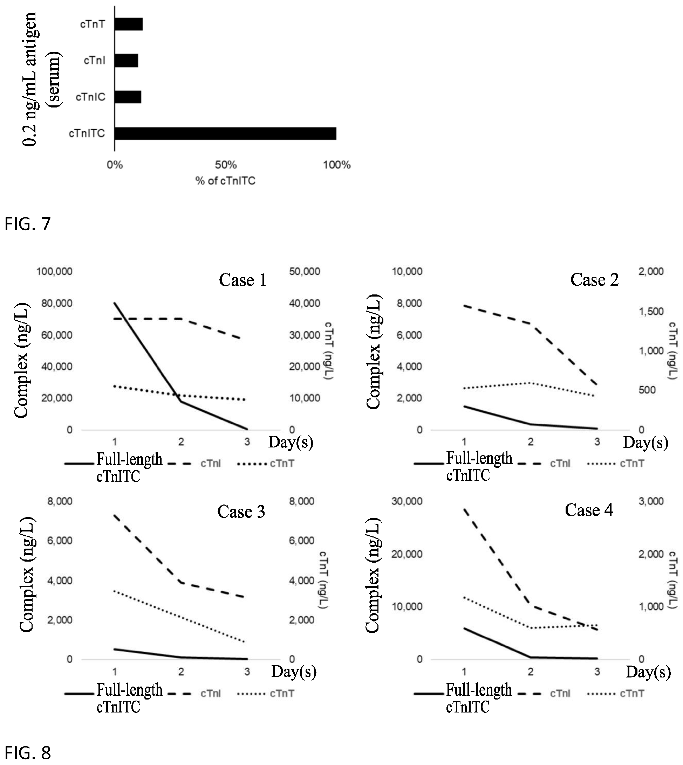

The specificity of detection kit 3a was analyzed. Equal concentrations of different antigens were spiked into the serum of healthy people, and the samples were separately analyzed using detection kit 3a by the chemiluminescent immunoassay method. The antigens analyzed included: cTnT (Hytest, 8RTT5), cTnl (Hytest, 8RT17), cTnIC (Hytest, 8ICR3) and cTnITC (Hytest, 8ITCR). The experimental results are shown in FIG. 7. Detection kit 3a was able to recognize only the cTnITC antigen, but was unable to recognize cTnT, cTnl or the binary cTnIC complex.

In summary, detection kit 3a can be used for the detection of clinical serum samples, while detection kit 3 demonstrates superior performance in terms of overall sensitivity and specificity.

### Example 7 Clinical Performance Evaluation of Large-size cTnlTC-Specific Immunoassay

Detection kit 3 was used for clinical performance evaluation. To evaluate the clinical performance of detecting large-size cTnITC using the sandwich immunoassay, dynamic monitoring was performed on serum samples from patients with myocardial injury at different time points. Meanwhile, the high-sensitivity cTnl and cTnT levels in the serum of patients were detected.

Patients with myocardial injury were enrolled, and the enrolled patients were aged 18 years or older. These patients were diagnosed with myocardial infarction or heart failure, or had undergone cardiac surgery, and exhibited elevated levels of high-sensitivity cTnl or high-sensitivity cTnT. After enrollment, heparin lithium plasma samples from the patients were collected for three consecutive days, and the concentrations of the large-size cTnITC in the samples were measured using a Mindray chemiluminescence analyzer and accompanying reagents.

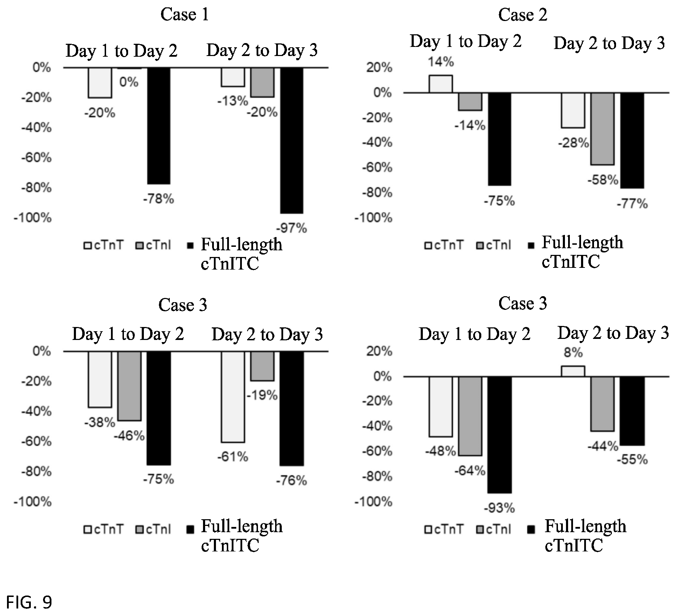

The results are shown in FIG. 8. The concentration of the large-size cTnITC exhibited a consistent trend of change with the high-sensitivity cTnl and cTnT levels, indicating that the concentration of the large-size cTnITC had clinical value and could be used for the evaluation of myocardial injury in patients. A further analysis was performed on the concentration changes of the large-size cTnITC, and high-sensitivity cTnl and cTnT. As shown in FIG. 9, the concentration of the large-size cTnITC exhibited a faster rate of change compared with cTnl and cTnT, indicating that the concentration changes of the large-size cTnITC were more sensitive to myocardial injury in patients.

Various modifications of the disclosure in addition to those described herein will be apparent to those skilled in the art from the foregoing description. Such modifications are also intended to fall within the scope of the appended claims. The full scope of the disclosure is given by the appended claims and any equivalents thereof.

## Claims

1. A composition for detecting a large-size cTIlTC in a sample, comprising a first group of antibodies and a second group of antibodies, wherein
the first group of antibodies comprises one or more antibody 1, each being independently selected from antibodies that specifically bind to any one segment of the amino acid sequence of positions 67-222 of cTnT (cardiac troponin T);
the second group of antibodies comprises one or more antibody 2, each being independently selected from antibodies that specifically bind to any one segment of the amino acid sequence of TnC (troponin C).

2. The composition of claim 1, wherein the first group of antibodies does not comprise an antibody that specifically binds to any one segment of the amino acid sequence of positions 223-287 of cTnT.

3. The composition of claim 1 or 2, wherein the second group of antibodies further comprises:
one or more antibody 3, each being independently selected from antibodies that specifically bind to cTnIC (cardiac troponin IC binary complex); and/or
one or more antibody 4, each being independently selected from antibodies that specifically bind to any one segment of the amino acid sequence of positions 18-210 of cTnl (cardiac troponin I).

4. The composition of any one of claims 1-3, wherein the first group of antibodies comprises one or more of the antibody 1, and the second group of antibodies comprises one or more of the antibody 2 and one or more of the antibody 3.

5. The composition of any one of claims 1-4, wherein each of the antibody 1 is independently selected from antibodies that specifically bind to amino acids at positions 67-86, positions 119-138, positions 132-151, positions 145-164 or positions 171-190 of cTnT;
preferably, each of the antibody 1 is independently selected from antibodies that specifically bind to amino acids at positions 119-138, positions 132-151 or positions 171-190 of cTnT.

6. The composition of any one of claims 3-5, wherein each of the antibody 4 is independently selected from antibodies that specifically bind to amino acids at positions 1-15, positions 13-22, positions 18-22, positions 18-28, positions 18-35, positions 22-31, positions 22-40, positions 23-29, positions 24-40, positions 25-40, positions 26-35, positions 34-37, positions 41-49, positions 83-89, positions 86-90, positions 87-90, positions 117-126, positions 130-145, positions 169-178, positions 186-192, positions 190-196 or positions 195-209 of cTnl;
preferably, each of the antibody 4 is independently selected from antibodies that specifically bind to amino acids at positions 22-40, positions 41-49 or positions 83-89 of cTnl;
more preferably, each of the antibody 4 is independently selected from antibodies that specifically bind to amino acids at positions 41-49 of cTnl.

7. A kit for detecting a large-size cTnITC in a sample, comprising a capture antibody and a detection antibody, wherein the capture antibody is selected from one group of a first group of antibodies and a second group of antibodies, and the detection antibody is selected from the other group of the first group of antibodies and the second group of antibodies, wherein
the first group of antibodies comprises one or more antibody 1, each being independently selected from antibodies that specifically bind to any one segment of the amino acid sequence of positions 67-222 of cTnT;
the second group of antibodies includes one or more antibody 2, each being independently selected from antibodies that specifically bind to any one segment of the amino acid sequence of TnC.

8. The kit of claim 7, wherein the first group of antibodies does not comprise an antibody that specifically binds to any one segment of the amino acid sequence of positions 223-287 of cTnT.

9. The kit of claim 7 or 8, wherein the second group of antibodies further comprises:
one or more antibody 3, each being independently selected from antibodies that specifically bind to cTnIC; and/or
one or more antibody 4, each being independently selected from antibodies that specifically bind to any one segment of the amino acid sequence of positions 18-210 of cTnl.

10. The kit of any one of claims 7-9, wherein the first group of antibodies comprises one or more of the antibody 1, and the second group of antibodies comprises one or more of the antibody 2 and one or more of the antibody 3.

11. The kit of any one of claims 7-11, wherein the capture antibody is the first group of antibodies and the detection antibody is the second group of antibodies.

12. The kit of any one of claims 7-11, wherein each of the antibody 1 is independently selected from antibodies that specifically bind to amino acids at positions 67-86, positions 119-138, positions 132-151, positions 145-164 or positions 171-190 of cTnT; preferably, each of the antibody 1 is independently selected from antibodies that specifically bind to amino acids at positions 119-138, positions 132-151 or positions 171-190 of cTnT; and/or
each of the antibody 4 is independently selected from antibodies that specifically bind to amino acids at positions 1-15, positions 13-22, positions 18-22, positions 18-28, positions 18-35, positions 22-31, positions 22-40, positions 23-29, positions 24-40, positions 25-40, positions 26-35, positions 34-37, positions 41-49, positions 83-89, positions 86-90, positions 87-90, positions 117-126, positions 130-145, positions 169-178, positions 186-192, positions 190-196 or positions 195-209 of cTnl; preferably, each of the antibody 4 is independently selected from antibodies that specifically bind to amino acids at positions 22-40, positions 41-49 or positions 83-89 of cTnl; more preferably, each of the antibody 4 is independently selected from antibodies that specifically bind to amino acids at positions 41-49 of cTnl.

13. A method for detecting a large-size cTnITC in a sample *in vitro,* comprising steps of:
obtaining a test sample;
contacting the test sample with a capture antibody to form an antibody-antigen complex;
contacting the antibody-antigen complex with a detection antibody bearing a detectable label to form an antibody-antigen-antibody complex; and
detecting the signal produced by the detectable label to determine the presence and/or content of the large-size cTnITC in the test sample,
wherein the capture antibody is selected from one group of a first group of antibodies and a second group of antibodies, and the detection antibody is selected from the other group of the first group of antibodies and the second group of antibodies:
the first group of antibodies comprises one or more antibody 1, each being independently selected from antibodies that specifically bind to any one segment of the amino acid sequence of positions 67-222 of cTnT;
the second group of antibodies comprises one or more antibody 2, each being independently selected from antibodies that specifically bind to any one segment of the amino acid sequence of TnC.

14. The method of claim 13, wherein the first group of antibodies does not comprise an antibody that specifically binds to any one segment of the amino acid sequence of positions 223-287 of cTnT.

15. The method of claim 13 or 14, wherein the second group of antibodies further comprises:
one or more antibody 3, each being independently selected from antibodies that specifically bind to cTnIC; and/or
one or more antibody 4, each being independently selected from antibodies that specifically bind to any one segment of the amino acid sequence of positions 18-210 of cTnT.

16. The method of claim 15, wherein the first group of antibodies comprises one or more of the antibody 1, and the second group of antibodies comprises one or more of the antibody 2 and one or more of the antibody 3.

17. The method of any one of claims 13-16, wherein the capture antibody is the first group of antibodies and the detection antibody is the second group of antibodies.

18. Use of the composition of any one of claims 1-6 in the preparation of a reagent for detecting a large-size cTnITC.

19. Use of the composition of any one of claims 1-6, the kit of any one of claims 7-12, or the method of any one of claims 13-17 in the *in-vitro* diagnosis of myocardial injury.

20. A method for diagnosing myocardial injury *in vitro,* comprising a step of detecting a large-size cTnITC in a sample from a subject using the composition of any one of claims 1-6, the kit of any one of claims 7-12, or the method of any one of claims 13-17.
